# EUROPEAN PATENT SPECIFICATION

(11) **EP 4 403 098 B1**
(45) Date of publication and mention of the grant of the patent: **12.08.2026**
(21) Application number: 24151674.9
(22) Date of filing: 12.01.2024
(51) Int. Cl.: A61B 5/00

(54) **ELECTRONIC DEVICE, SLEEP STATE DETERMINATION METHOD, AND PROGRAM**
ELEKTRONISCHE VORRICHTUNG, SCHLAFZUSTANDSBESTIMMUNGSVERFAHREN UND PROGRAMM
DISPOSITIF ÉLECTRONIQUE, PROCÉDÉ DE DÉTERMINATION D'ÉTAT DE SOMMEIL ET PROGRAMME

(30) Priority: 19.01.2023 JP 2023006427
(43) Date of publication of application: 24.07.2024
(73) Proprietor: Casio Computer Co., Ltd., Tokyo 151-8543 (JP)
(72) Inventor: AOYAMA, Kotaro, Tokyo, 205-8555 (JP); NAKAGOME, Kouichi, Tokyo, 205-8555 (JP)
(74) Representative: Plougmann Vingtoft a/s

(56) References cited:
- CN-A- 113 704 048
- US-A1- 2007 282 215
- US-A1- 2021 090 740
- US-A1- 2021 338 152

## Description

The present disclosure relates generally to an electronic device, a sleep state determination method, and a program.

In the related art, devices are known that detect a sleep state of a person and attempt to wake up that person in a period in which the sleep state is light (REM phase). An example of such a device is disclosed in Japanese Unexamined Patent Application No. 2007-105111. This device includes a body motion sensor that measures the body motion of the sleeper, derives the heart rate per minute from signals from the body motion sensor, and detects the period in which the sleep state is light on the basis of the derived heart rate.

US 2007/282215 A1 discloses devices and methods for detecting disordered breathing involve determining that the patient is asleep and sensing one or more signals associated with disordered breathing indicative of sleep-disordered breathing while the patient is asleep. Sleep-disordered breathing is detected using the sensed signals associated with disordered breathing. The sensed signals associated with disordered breathing may also be used to acquire a respiration pattern of one or more respiration cycles. Characteristics of the respiration pattern are determined. The respiration pattern is classified as a disordered breathing episode based on the characteristics of the respiration pattern. One or more processes involved in the detection of disordered breathing are performed using an implantable device.

US 2021/338152 A1 discloses systems and methods for use in monitoring one or more physiological parameters of a user. The system comprises: at least one sensing unit comprising at least one vibration sensor; and at least one elastic tube configured to be embedded within a cushioning layer for supporting at least a portion of the user. One end of the at least one tube is sealed and the other end of the at least one tube is closed by the at least one sensing unit so as to form a volume that is filled with fluid and that is defined by one or more inner walls of the tube and a surface of the at least one sensing unit. The at least one elastic tube is configured to transmit vibrations from one or more sections of the at least one tube to the at least one sensing unit for detection by the at least one vibration sensor.

Thus, with conventional devices, the period in which the sleep state is light is detected on the basis of the heart rate and, consequently, the sleep state cannot be correctly detected when the heartbeat cannot be correctly detected.

The invention is set out in the appended set of claims.

A more complete understanding of this application can be obtained when the following detailed description is considered in conjunction with the following drawings, in which:
FIG. 1 is a drawing illustrating a configuration example of an electronic device according to Embodiment 1;
FIG. 2 is a drawing illustrating an example of a pulse wave;
FIG. 3 is a drawing for explaining an analysis period and a determination period;
FIG. 4 is a drawing illustrating an example of a graph of a heart rate;
FIG. 5 is a drawing illustrating an example of the graph of the heart rate after a Min filter applied;
FIG. 6 is a drawing for explaining the operations of the Min filter;
FIG. 7 is a drawing for explaining a histogram of a standard deviation of the heart rate;
FIG. 8 is a drawing for explaining heart rate data used in the deriving of a heart rate threshold;
FIG. 9 is a drawing for explaining a timing at which an alarm starts;
FIG. 10 is a drawing for explaining a case in which the heart rate suddenly becomes a low value;
FIG. 11 is a drawing for explaining a method of acquiring the heart rate data used in the deriving of the heart rate threshold, without using a histogram;
FIG. 12 is a flowchart of analysis processing according to Embodiment 1; and
FIG. 13 is a flowchart of determination processing according to Embodiment 1.

Hereinafter, embodiments are described while referencing the drawings. Note that, in the drawings, identical or corresponding components are denoted with the same reference numerals.

### Embodiment 1

In one example, an electronic device 100 according to Embodiment 1 is a smartwatch. The electronic device 100 acquires, by built-in sensors, biological signals of a user, estimates a sleep state of the user, determines a timing at which sleep is light as a recommended wake-up time, and wakes up the user by an electronic sound and/or vibration.

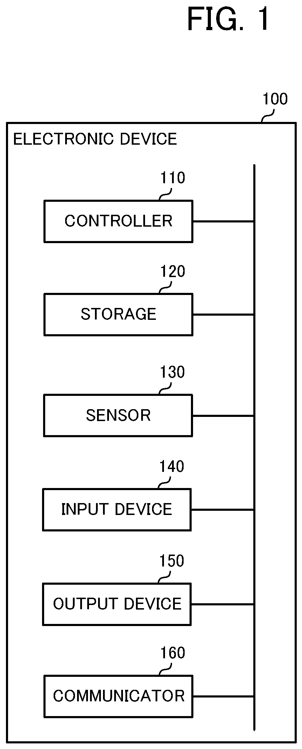

As illustrated in FIG. 1, the electronic device 100 includes, as functional components, a controller 110, a storage 120, a sensor 130, an input device 140, an output device 150, and a communicator 160.

The controller 110 is configured from a processor such as a central processing unit (CPU) or the like, and executes various processings described later by executing programs stored in the storage 120. Note that the controller 110 is compatible with multithreading functionality, in which a plurality of processings are executed in parallel. As such, the controller 110 can execute the various processings described below in parallel. Additionally, the controller 110 is provided with a clock function and a timer function, and can measure the date and time, and the like.

The storage 120 is configured from read-only memory (ROM), flash memory, random access memory (RAM), or the like. Programs to be executed by the CPU of the controller 110, and data needed in advance to execute these programs are stored in the ROM. The flash memory is writable non-volatile memory, and stores data that is desired to be retained even after the power is turned OFF. Data that is created or modified during the execution of the programs is stored in the RAM.

The sensor 130 includes a pulse wave sensor for detecting a plethysmogram of the user, and an acceleration sensor for detecting body motion of the user.

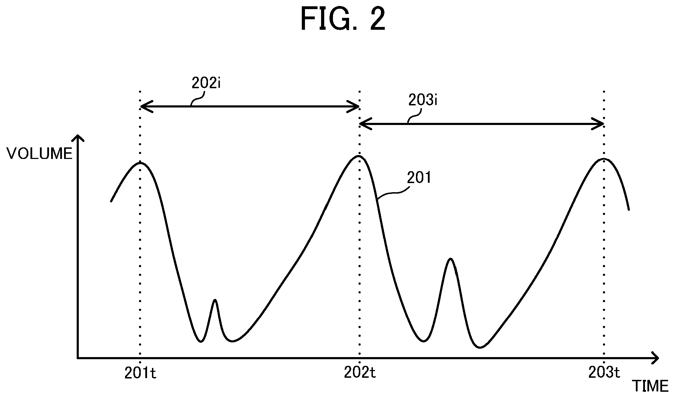

The pulse wave sensor detects, at a certain sampling frequency (for example, 250 Hz) changes in the amount of light absorbed by oxyhemoglobin in the blood by irradiating light from the surface of the skin of the user and measuring the reflected light and the transmitted light thereof. These changes in the amount of absorbed light correspond to changes in blood vessel volume. As such, the pulse wave sensor obtains a pulse wave 201 that captures changes in blood vessel volume as a waveform, as illustrated in FIG. 2.

Moreover, since it is thought that changes in blood vessel volume are synchronized with the beating of the heart, it is possible to estimate the timing at which the amplitude of the pulse wave peaks as the timing when a beat of the heart occurs (hereinafter referred to as "beat timing"). Additionally, it is possible to estimate, as a heartbeat interval (R-R interval, "RRI"), a time interval between two adjacent peaks of the pulse wave on the time axis.

In FIG. 2, the timings at which the amplitude of the pulse wave 201 peaks correspond to beat timings 201t, 202t, 203t, and the intervals between the peaks of the pulse wave 201 correspond to RRI 202i and RRI 203i. Moreover, when the unit of the RRI is seconds, the controller 110 can calculate the heart rate (HR) per minute by dividing 60 seconds by the RRI. In the present embodiment, the controller 110 calculates, one time per second, the heart rate per minute on the basis of the pulse wave detected by the sensor 130.

Additionally, the sensor 130 includes the acceleration sensor, and the acceleration sensor detects acceleration in the direction of each of three axes (mutually orthogonal X axis, Y axis, and Z axis) to detect the body motion of the user. In the present embodiment, the controller 110 samples detection values of the acceleration sensor at a certain sampling frequency (for example, 32 Hz) to acquire an acceleration data string.

In one example, the input device 140 is constituted by a push button switch, a touch panel, or the like. In one example, the input device 140 is a user interface for receiving user operations such as setting of an alarm time (expected wake up time), setting of alarm operation content (electronic sound only, vibration only, electronic sound + vibration, or the like), ON/OFF of the alarm function, and the like.

In one example, the output device 150 includes a display such as a liquid crystal display or an organic electroluminescence (EL) display, a sound outputter such as a speaker or a buzzer, a vibration device such as a vibrator, and the like. However, a configuration is possible in which the output device 150 includes only a portion of these components. In one example, the display of the output device 150 displays a current time and the like. When the electronic device 100 is used as an alarm clock, the output device 150 emits a sound at the recommended wake-up time, for example.

The communicator 160 is a communication interface for exchanging data and the like with other external devices. This communication interface may be wireless or wired. In one example, the electronic device 100 is capable of sending, via the communicator 160, the determined recommended wake-up time to an external server or the like.

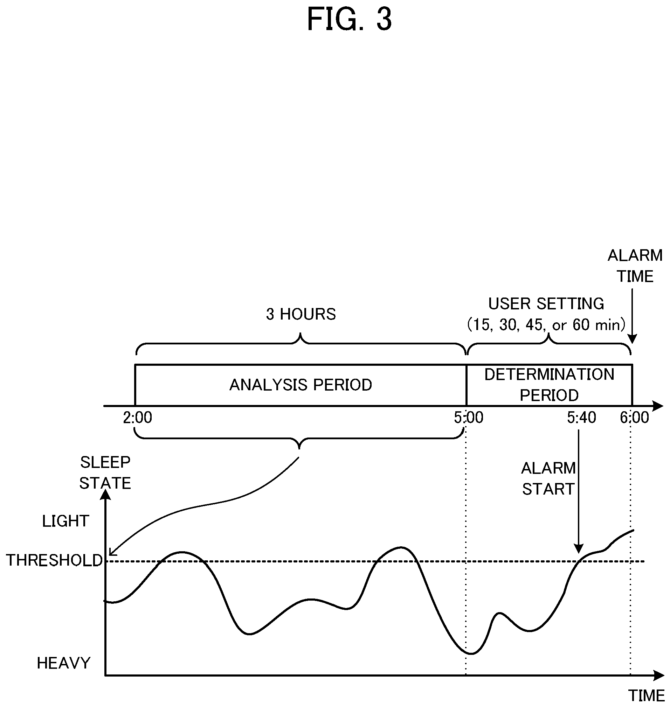

As illustrated in FIG. 3, in an analysis period (first period) while the user is sleeping, the electronic device 100 acquires and statistically processes a plurality of biological signals (first period data) of the user, and derives a threshold for determining whether the sleep is light. Then, in a determination period (second period) immediately before the set alarm time, the electronic device 100 acquires the biological signals (second period data) of the user, estimates the sleep state of the user on the basis of the threshold, determines a timing at which the sleep becomes light (timing at which a value of the second period data exceeds the threshold) as the recommended wake-up time, and starts an alarm (emits the electronic sound, operates the vibrator, or the like). In the present embodiment, the analysis period is three hours, and the determination period can be selected by the user from any of 15, 30, 45, and 60 minutes.

In the example illustrated in FIG. 3, the alarm time is set to 06:00 and the determination period is set to 60 minutes. In this case, from 02:00 to 05:00 is the analysis period, and from 05:00 to 06:00 is the determination period. Moreover, in the determination period, the timing at which the sleep state becomes lighter than the threshold is 05:40 and, as such, the alarm is started at that timing.

The inventors conducted tests on a plurality of subjects in which sleep stage measurement using a simple electroencephalogram, heart rate measurement, and acceleration measurement were performed simultaneously, and obtained the following findings from analyses of the test results.
(a) The heart rate is high when the sleep state is light, and is low when the sleep state is not light (state of deep sleep or state of medium sleep).
(b) The standard deviation of the heart rate is great when the sleep state is light, and is small when the sleep state is not light.
(c) The acceleration is great when the sleep state is light, and is small when the sleep state is not light.
(d) The heart rate, the standard deviation of the heart rate, and the acceleration not only differ by subject, but also have slight differences day by day, even for the same subject.

In the present embodiment, the effects of individual differences and day to day differences are suppressed by deriving a determination threshold using the biological signal data acquired in the analysis period immediately before the determination period in which the determination of the recommended wake-up time is performed.

The heart rate acquired by the controller 110 frequently includes outliers due to errors of the values detected by the sensor 130, errors when calculating the heart rate from the pulse wave, and the like.

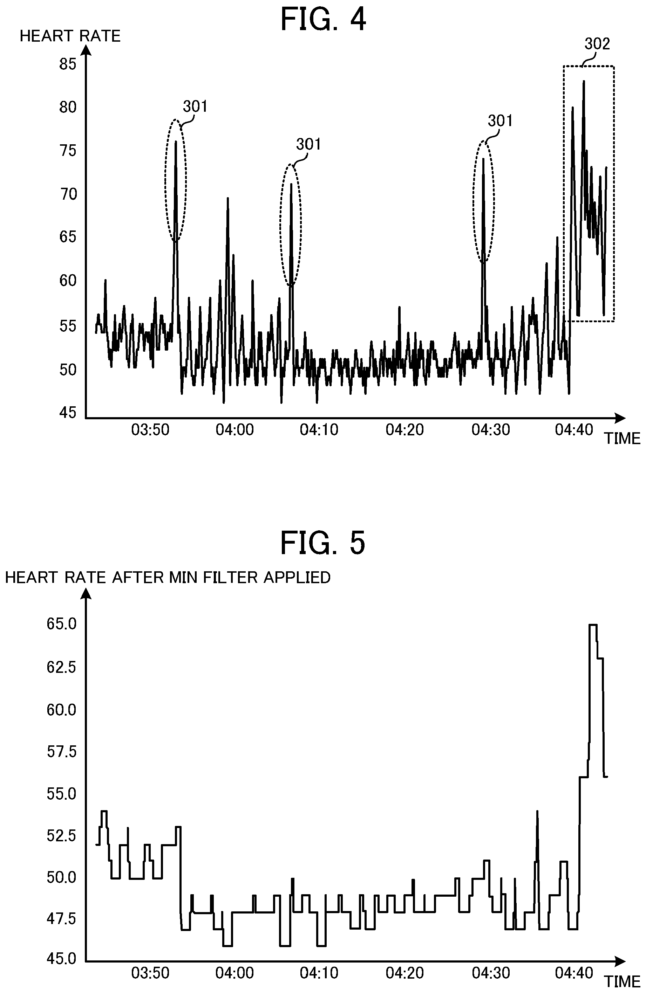

For example, FIG. 4 illustrates an example of the heart rate of a certain person from about 03:45 to about 04:45 on a certain day. In FIG. 4, the portions where the heart rate exceeds 70 are portions at which the value increases suddenly, and are thought to be outliers. Such outliers occur, for example, when the algorithm for calculating the heart rate from the pulse wave erroneously recognizes a timing, that is not a peak of the pulse wave, as a peak. Conversely, if the algorithm misses the peak of the pulse wave, an outlier at which the heart rate suddenly decreases will be obtained. However, it is possible to suppress, to the greatest extent possible, outliers at which the heart rate suddenly drops by adopting an algorithm that minimizes missing of the peaks of the pulse wave.

In reality, there are a variety of other causes of outliers but, in FIG. 4, there are, as representative outliers, suddenly occurring outliers as seen in the portions surrounded by the dotted oval 301, and continuously occurring outliers as seen in the portion surrounded by the dotted rectangle 302. In the present embodiment, outlier removal using a filter (for example, a Min filter to be described later) is performed to remove the suddenly occurring outliers, and outlier removal using standard deviation is performed to remove the continuously occurring outliers.

In the present embodiment, an algorithm is adopted that avoids missing pulse wave peaks as much as possible when calculating the heart rate from the pulse wave. In this case, the possibility of outliers at which the heart rate becomes greater than expected values occurring increases but, conversely, the possibility of outliers at which the heart rate becomes smaller than expected values occurring decreases significantly. Accordingly, it is possible to substantially remove the outliers by correcting the heart rate data using a Min filter that replaces the heart rate with the minimum value within the most recent predetermined period (window).

For example, when the Min filter is applied to the graph of the heart rate illustrated in FIG. 4, a graph of a heart rate such as illustrated in FIG .5 is obtained. Comparing FIGS. 4 and 5, it can be confirmed that there are no particular outliers in FIG. 5, even at the timings of the outliers of heart rates of 70 or greater that occur in the portions surrounded by the dotted ovals 301 of FIG. 4.

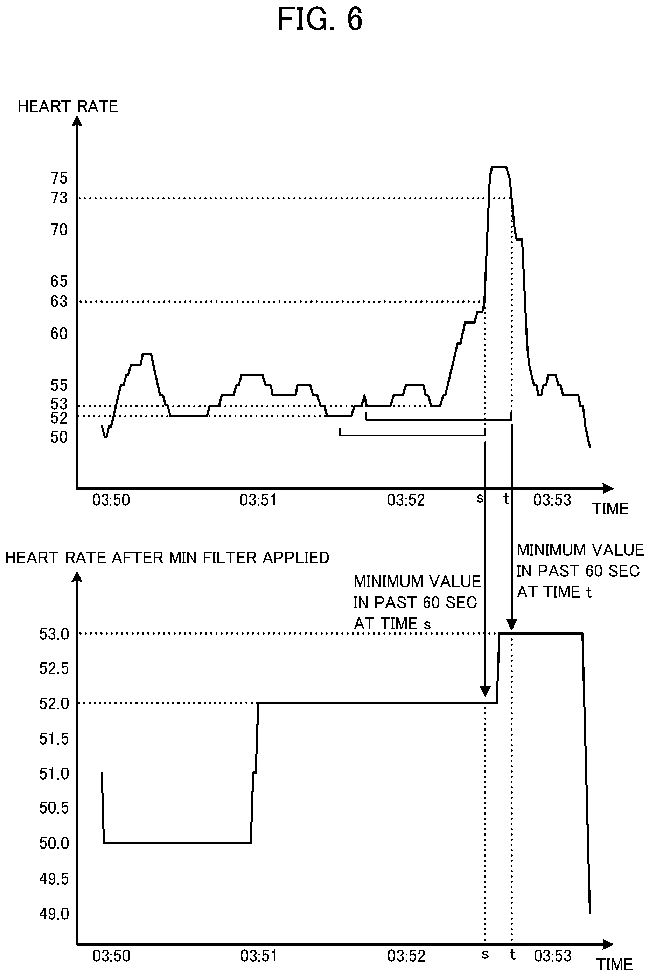

Next, the Min filter is described in detail while referencing FIG. 6. FIG. 6 is a drawing obtained by enlarging from about 03:50 to 03:53 in FIGS. 4 and 5. The Min filter is a filter that replaces each heart rate with the minimum value of the heart rate within a most recent certain period (first window period). Note that, in the present embodiment, the first window period is set to 60 seconds. For example, in FIG. 6, the original heart rate at the point in time of a time t is 73, but the minimum value of the heart rate within the first window period of from 60 seconds before the time t to the time t is 53. As such, the heart rate at the time t after the Min filter applied is 53. Likewise, in FIG. 6, the original heart rate at the point in time of a time s is 63, but the minimum value of the heart rate within the first window period of from 60 seconds before the time s to the time s is 52. As such, the heart rate at the time s after the Min filter applied is 52.

Thus, the Min filter is a filter that replaces the heart rate at each time with the minimum value within the period of a window (first window) having a length of the first window period. Moreover, in the analysis period (the first period), the controller 110 applies the Min filter to each of the plurality of acquired heart rate data (the first period data) while temporally shifting this first window a little at a time (for example, sliding one second at a time) to generate a heart rate data group (first corrected data group) that is corrected such that the suddenly occurring outliers are removed. Note that, as described later, in the determination period (the second period) as well, the controller 110 applies the Min filter to each of the plurality of acquired heart rate data (the second period data) while temporally shifting this first window a little at a time (for example, sliding one second at a time) to generate a heart rate data group (second corrected data group) that is corrected such that the suddenly occurring outliers are removed.

Additionally, the inventors discovered that, when the heart rate data is correct, the standard deviation thereof falls within a certain (predetermined) range. Firstly, when the standard deviation is excessively small, it can be said to be mechanical data without biological fluctuations, and it is understood that such heart rate data contains some sort of error. Moreover, there is a limit to the magnitude of heart rate fluctuation of a normal person and, as such, when the standard deviation is excessively large, it is understood that normal data is not being acquired. As such, when the heart rate is a normal heart rate, the standard deviation thereof falls within a predetermined range. Accordingly, when the standard deviation of the heart rate does not fall within the predetermined range, the controller 110 can determine that the heart rate is not being correctly acquired.

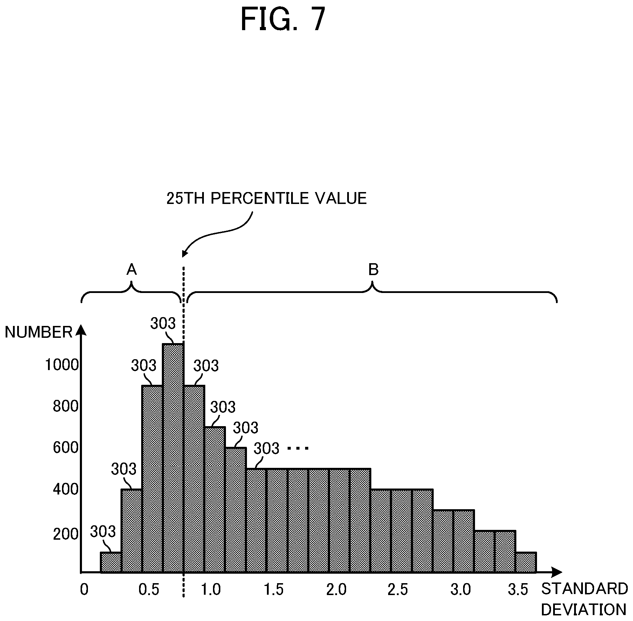

However, as described above, there is a possibility that the heart rate acquired in the present embodiment contains outliers, and when outliers are contained, the standard deviation becomes greater than the expected values. Therefore, in the present embodiment, the controller 110 is configured to create a histogram of the standard deviations of the heart rate, and only use data of the standard deviations less than or equal to the 25th percentile value (first quartile) in the determination of the recommended wake-up time. Note that, here, as illustrated in FIG. 7, the histogram is expressed by bins 303 in which the number of data included in the range of values of the horizontal axis (the standard deviation in Figure 7) is plotted on the vertical axis.

Specifically, the controller 110 slides the window (the second window) of the second window period (in the present embodiment, 30 seconds) one second at a time with respect to the heart rate data to calculate the standard deviation for every piece of data included in that second window. As a result, 10,800 standard deviations are obtained in the analysis period (three hours = 180 minutes = 10,800 seconds) and, when creating the histogram, a histogram such as that illustrated in FIG. 7, for example, is obtained. Here, when calculating the 25th percentile value of the standard deviation, in the example of FIG. 7, a value of approximately 0.833 is obtained. The controller 110 extracts the data (the heart rate data after the Min filter applied) in the range of A that corresponds to the standard deviations less than or equal to the 25th percentile value. Moreover, this extracted data (extracted corresponding data) is used in the analysis, and the data of the range of B that corresponds to the standard deviations that exceed the 25th percentile value is set as error values and is not used in the analysis.

When calculating the standard deviation of the data within the second window in the continuously occurring outlier portion (for example, in FIG. 4, the portion surrounded by the dotted rectangle 302), that standard deviation becomes greater than the standard deviation of the other portions (exceeds the 25th percentile value) and, as such, the continuously occurring outliers can be removed by using the data of the range less than or equal to the 25th percentile value (the range of A of FIG. 7). Thus, the controller 110 uses the standard deviations to generate a standard deviation data group (data Dn of the range of A) to be used in the analysis processing described later.

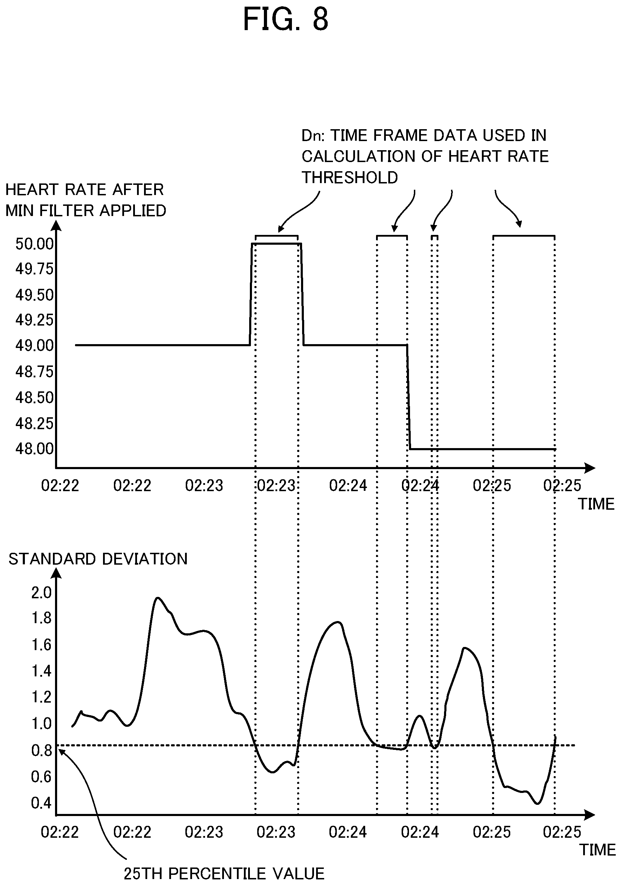

Then, as illustrated in FIG. 8, the controller 110 uses data Dn of the heart rate (the heart rate after the Min filter applied) in the time window in which the standard deviation is less than or equal to the 25th percentile value to derive, using Equation (1) below, a heart rate threshold HR_TH (heart rate threshold for determining whether the sleep state is light). $HR_TH = Dn_ave + h \times Dn_std$

The various values in Equation (1) are defined as follows:
Dn_ave = Average of all Dn data
Dn_std = Standard deviation of all Dn data
h = Adjustment factor (in the present embodiment, "2").

Additional description of Equation (1) is given below. It is thought that the sleep state of the user is light when the heart rate is a certain degree greater than the average (Dn_ave). Specifically, it is clear that it is sufficient that the heart rate threshold is set to the average + α. However, the magnitude of heart rate fluctuation varies from person to person, and for people with great fluctuation, the value of α must be set to a greater value so that the heart rate threshold is not easily exceeded. Moreover, when the heart rate fluctuation is great, the standard deviation (Dn_std) increases. Accordingly, in Equation (1) described above, a more reliable heart rate threshold (capable of absorbing differences in the heart rate from person to person) is set by adding h×Dn_std to Dn_ave.

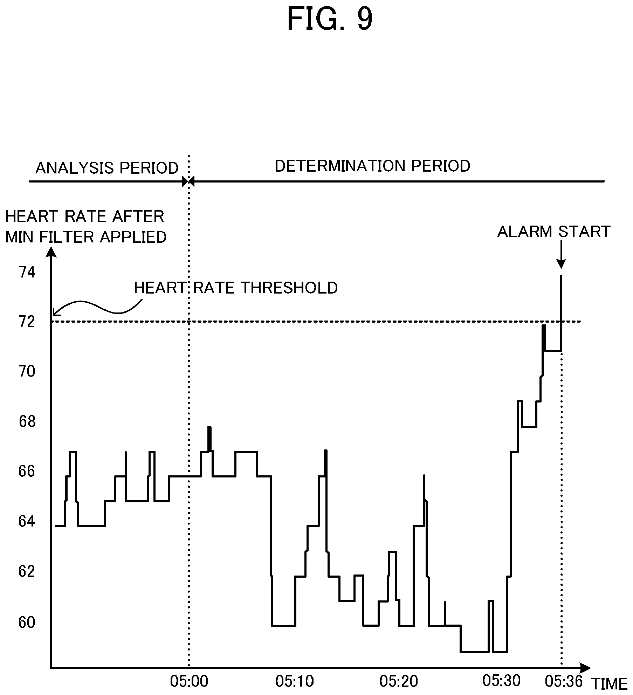

Then, when the heart rate after the Min filter applied in the determination period becomes greater than the heart rate threshold HR_TH derived by Equation (1), the controller 110 determines that it is the recommended wake-up time, starts the alarm, and wakes up the user. For example, FIG. 9 illustrates a graph of the heart rate after the Min filter applied of a certain evening of a certain user. Here, when the heart rate threshold HR_TH is 72 and the determination period starts at 05:00, the controller 110 determines that the recommended wake-up time is 05:36, which is when the heart rate after the Min filter applied, after 5:00, becomes greater than 72, and starts the alarm.

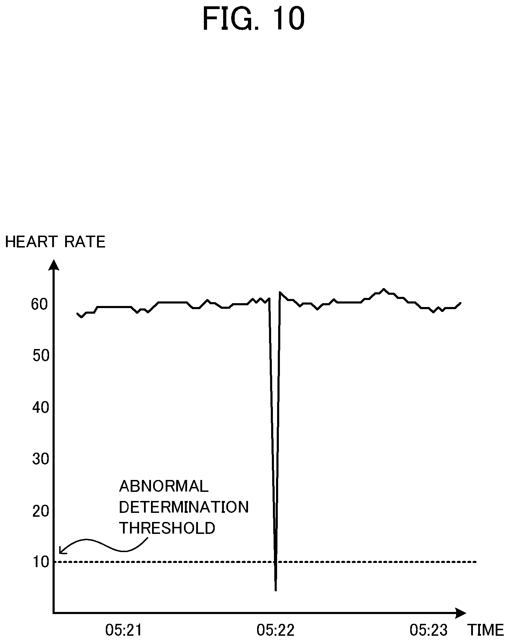

However, while extremely rare, there are, for example, cases in which the heart rate suddenly becomes an abnormally small outlier, such as the value of the heart rate at the time 05:22 illustrated in FIG. 10. In such a case, for the time from 05:21 to 05:23, the heart rate after the Min filter applied also becomes this outlier, and correct determination of the sleep state on the basis of the heart rate threshold is impossible. As such, the controller 110 is configured to perform the determination of the sleep state using the acceleration threshold instead of the heart rate when the heart rate after the Min filter applied is less than or equal to an abnormal determination threshold (for example, 10).

The acceleration threshold is derived by the controller 110 on the basis of the acceleration during the analysis period. In the present embodiment, the controller 110 derives an acceleration threshold ACC_TH in accordance with Equation (2) below, from an acceleration average (ACC_ave) and an acceleration standard deviation (ACC_std) during the analysis period. $ACC_TH = ACC_ave + k \times ACC_std$

The various values in Equation (2) are defined as follows:
ACC_ave = Average of all acceleration data during the analysis period
ACC_std = Standard deviation of all acceleration data during the analysis period
k = Adjustment factor (in the present embodiment, "8").

Additional description of Equation (2) is given below. It is thought that the sleep state of the user is light when the acceleration is a certain degree greater than the average (ACC_ave). Specifically, it is clear that it is sufficient that the acceleration threshold is set to the average + α. However, the magnitude of acceleration fluctuation varies from person to person, and for people with great fluctuation, the value of α must be set to a greater value so that the acceleration threshold is not easily exceeded. Moreover, when the acceleration fluctuation is great, the standard deviation (ACC_std) increases. Accordingly, in Equation (2) described above, a more reliable acceleration threshold (capable of absorbing the differences in the acceleration from person to person) is set by adding k×ACC_std to ACC_ave.

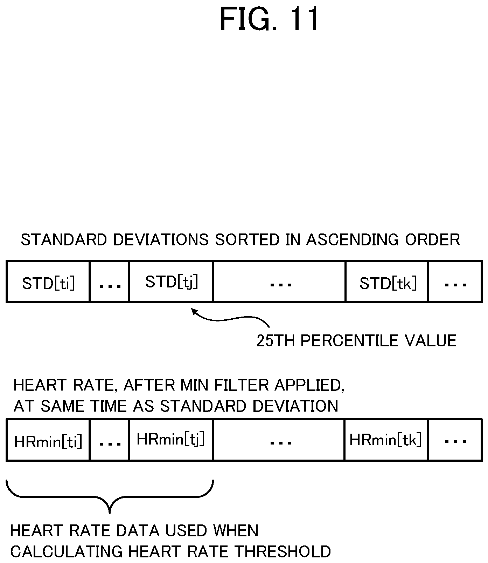

Note that, when deriving the heart rate threshold, the controller 110 can calculate the 25th percentile value without creating the histogram of the standard deviations of the heart rate. In one example, as illustrated in FIG. 11, when the controller 110 sorts the standard deviations (STD[ti], STD[tj], STD[tk], ...) of the heart rate at each time (ti, tj, tk, ...) in ascending order, the value corresponding to 1/4 of the total number from the smallest value is the 25th percentile value. Accordingly, the heart rate threshold may be derived by obtaining the average (Dn_ave) and the standard deviation (DN_std) for the heart rate data after the Min filter applied (HRmin[ti], ..., HRmin[tj]) that correspond to the times (in this example, ti, ..., tj) of the standard deviations from the smallest value to that value.

Additionally, it is sufficient that the heart rate threshold (the first threshold) is derived by performing some sort of statistical processing on the heart rate data, and it is not absolutely necessary to use the 25th percentile value of the standard deviations of the heart rate. For example, a median of the standard deviations of the heart rate may be used, or an xth percentile value of the standard deviations of the heart rate may be used for a desired x (where x is from 0 to 100). Furthermore, in Equation (1), Dn_med (median of all Dn data) or Dn_mode (mode obtained from all Dn data) may be used instead of Dn_ave (average of all Dn data). Additionally, in Equation (1), a variance of all Dn data or a predetermined value may be used instead of Dn_std (standard deviation of all Dn data).

Likewise, it is sufficient that the acceleration threshold (the second threshold) is derived by performing some sort of statistical processing on the acceleration data. For example, in Equation (2), ACC_med (median of all acceleration data during analysis period) or ACC_mode (mode obtained from all acceleration data during analysis period) may be used instead of ACC_ave (average of all acceleration data during the analysis period). Additionally, in Equation (2), a variance of all of the acceleration data during the analysis period or a predetermined value may be used instead of ACC_std (standard deviation of all acceleration data during the analysis period).

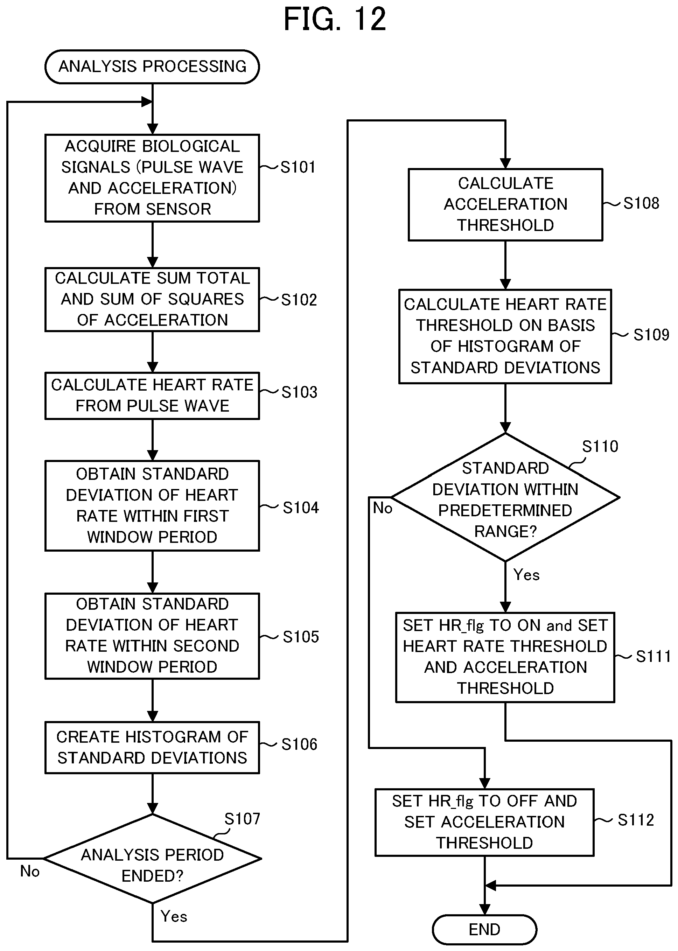

Next, the analysis processing executed in the analysis period is described while referencing FIG. 12. The analysis processing starts when the time becomes the analysis period, and when the analysis period ends, the first threshold and the second threshold are set and the analysis processing is ended. In one example, when the alarm time is set to 06:00 and the determination period is set to 60 minutes, as illustrated in FIG. 3, the analysis period is from 02:00 to 05:00 and, as such, the analysis processing starts when the time is 02:00.

Firstly, the controller 110 acquires biological signals from the sensor 130 (step S101). More specifically, the controller 110 acquires the pulse wave using the pulse wave sensor, and the acceleration using the acceleration sensor.

Next, the controller 110 calculates a sum total and a sum of squares of the acceleration acquired in step S101 (step S102). Specifically, when the sum total of the acceleration is stored in a variable SA, the sum of squares is stored in a variable TA, and the acceleration acquired in step S101 is stored in a variable A, in step S102, it is sufficient that A is added to the variable SA, and the square of A is added to TA. By successively calculating the sum total and the sum of squares of the acceleration in this manner, it is possible to perform the derivation of the acceleration threshold in step S108, described layer, in a short amount of time.

Next, the controller 110 calculates the heart rate from the pulse wave acquired in step S101 (step S103). The heartbeat interval (sec) can be obtained simply from the pulse wave, and the heart rate can be calculated by dividing 60 by the heartbeat interval. However, in the present embodiment, in order to minimize outliers as much as possible, it is desirable that the heart rate be calculated using an algorithm that avoids missing the peaks of the pulse wave as much as possible.

Next, the controller 110 obtains the minimum value of the heart rate within the most recent first window period (step S104). Note that the first window period is one minute in the present embodiment, but may be set to a shorter period (for example, 30 seconds) or to a longer period (for example, 2 minutes). Setting the first window period to a shorter period makes it easier to follow changes in the heart rate and, in such a case, the alarm can be started immediately at the timing at which the sleep of the user becomes light. Meanwhile, setting the first window period to a longer period makes it possible to suppress the effects of outliers of the heart rate to a greater degree.

Next, the controller 110 obtains the standard deviations of the heart rate within the most recent second window period (step S105). The second window period is 30 seconds in the present embodiment, but may be set to a shorter period (for example, 15 seconds) or to a longer period (for example, 1 minute). Whether the heart rate calculated in step S103 is a normal value is determined on the basis of the standard deviations obtained here. Accordingly, setting the second window period to a shorter period makes it possible to immediately detect that the heart rate is a value that is not normal. Meanwhile, setting the second window period to a longer period makes it possible to more correctly determine whether the heart rate is a normal value.

Next, the controller 110 creates the histogram of the standard deviations obtained in step S105 (step S106). At this time, the controller 110 calculates, for every bin of the histogram described above, the sum total and the sum of squares of the minimum value of the heart rate obtained in step S104. By successively calculating, for every bin of the histogram described above, the sum total and the sum of squares of the minimum value of the heart rate in this manner, it is possible to perform the derivation of the heart rate threshold in step S109, described layer, in a short amount of time.

Next, the controller 110 determines whether the analysis period is ended (step S107). When the analysis period is not ended (step S107; No), the controller 110 executes step S101.

When the analysis period is ended (step S107; Yes), the controller 110 derives the acceleration threshold using Equation (2) described above (step S108).

Next, the controller 110 calculates the 25th percentile value for the histogram created in step S106, and derives the heart rate threshold using Equation (1) described above (step S109).

Next, the controller 110 determines whether the standard deviation (Dn_std: standard deviation of all DN data) calculated in step S105 is within a predetermined range (step S110). When the standard deviation is within the predetermined range (step S110; Yes), the controller 110 sets a flag variable HR_flg to ON, sets so as to use both the heart rate threshold and the acceleration threshold in the determination processing described later (step S111), and ends the analysis processing.

Meanwhile, when the standard deviation is not within the predetermined range (step S110; No), the controller 110 sets the flag variable HR_flg to OFF, sets so as to use only the acceleration threshold in the determination processing described later (step S112), and ends the analysis processing. The determination condition in step S110 is one condition for determining whether the heart rate is correctly acquired in the analysis period and, as such, is also called a "first condition."

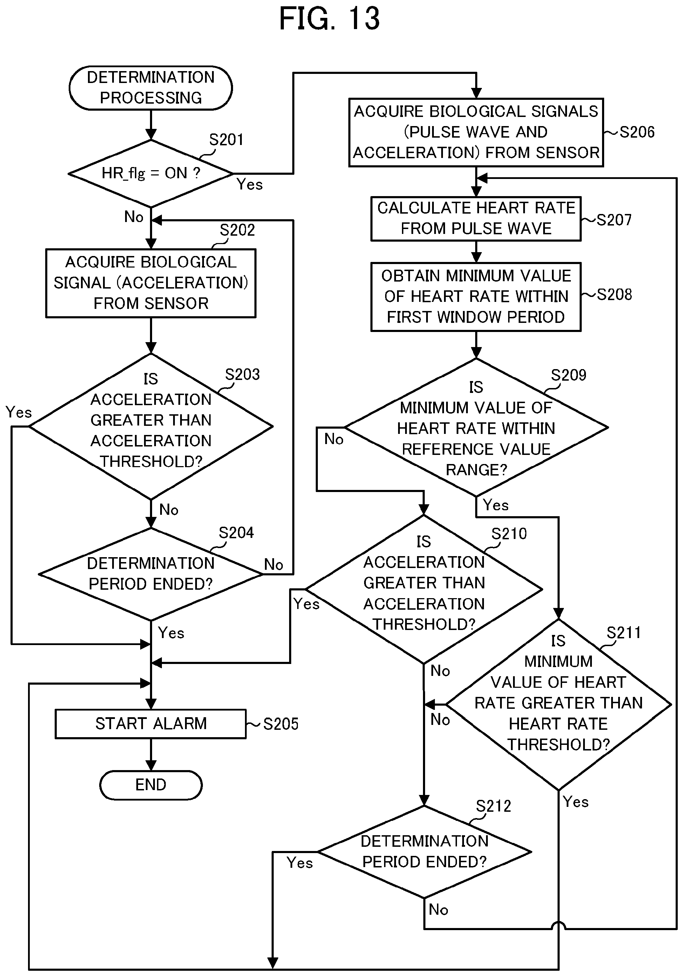

Next, the determination processing executed in the determination period is described while referencing FIG. 13. The determination processing starts when the time becomes the determination period, acquires determination data (the heart rate, the acceleration) at a predetermined time interval (for example, every one second for the heart rate, and every 1/32 second for the acceleration), and determines the sleep state of the user on the basis of the thresholds set in the analysis processing (the first threshold and the second threshold). In one example, when the alarm time is set to 06:00 and the determination period is set to 60 minutes, as illustrated in FIG. 3, the determination period is from 05:00 to 06:00 and, as such, the determination processing starts when the time is 05:00.

Firstly, the controller 110 determines whether the flag variable HR_flg is ON (step S201). However, when the flag variable HR_flg is not set to either ON or OFF (when the analysis processing is not ended), the controller 110 waits until the flag variable HR_flg is set to ON or OFF (until the analysis processing ends), and performs the processing of step S201 after the flag variable HR_flg is set to ON or OFF.

When the flag variable HR_flg is not ON (step S201; No), the controller 110 acquires the acceleration from the acceleration sensor of the sensor 130 (step S202). Then, the controller 110 determines whether the acceleration acquired in step S202 is greater than the acceleration threshold set in the analysis processing (step S203).

When the acceleration is greater than the acceleration threshold (step S203; Yes), the controller 110 determines that the current time is the recommended wake-up time that is the timing at which the sleep state is light, starts the alarm (step S205), and ends the determination processing.

When the acceleration is less than or equal to the acceleration threshold (step S203; No), the controller 110 determines whether the determination period is ended (step S204). When the determination period is not ended (step S204; No), step S202 is executed. When the determination period is ended (step S204; Yes), the time is the set alarm time and, as such, the controller 110 executes step S205 (alarm start).

Meanwhile, in step S201, when the flag variable HR_flg is ON (step S201; Yes), the controller 110 acquires the pulse wave using the pulse wave sensor, and acquires the acceleration using the acceleration sensor (step S206).

Next, the controller 110 calculates the heart rate from the pulse wave acquired in step S206 (step S207), and obtains, by the Min filter, the minimum value of the heart rate within the most recent first window period (step S208).

Next, the controller 110 determines whether the minimum value of the heart rate obtained in step S208 is within a predetermined reference value range (whether greater than the abnormal determination threshold (for example, 10)) (step S209). Note that, in the present embodiment, as in the analysis processing in the analysis period, the Min filter is applied while sliding the first window every one second in the determination processing in the determination period to obtain the minimum value of the heart rate within the most recent first window period. Accordingly, the determination of step S209 is performed every second. When the minimum value of the heart rate is outside the predetermined reference value range (less than or equal to the abnormal determination threshold) (step S209; No), the controller 110 determines, until calculating the next heart rate, whether the acceleration acquired in step S206 is greater than the acceleration threshold set in the analysis processing (step S210).

When the acceleration is greater than the acceleration threshold (step S210; Yes), the controller 110 determines that the current time that is the timing at which the sleep state is light is the recommended wake-up time, and executes step S205 (alarm start). When the acceleration is less than or equal to the acceleration threshold (step S210; No), the controller 110 determines whether the determination period is ended (step S212). When the determination period is not ended (step S212; No), step S206 is executed. When the determination period is ended (step S212; Yes), the time is the set alarm time and, as such, the controller 110 executes step S205 (alarm start).

Meanwhile, when, in step S209, the minimum value of the heart rate is within the predetermined reference value range (is greater than the abnormal determination threshold) (step S209; Yes), the controller 110 determines whether the minimum value of the heart rate obtained in step S208 is greater than the heart rate threshold set in the analysis processing (step S211).

When the minimum value of the heart rate is less than or equal to the heart rate threshold (step S211; No), step S212 is executed. Meanwhile, when the minimum value of the heart rate is greater than the heart rate threshold (step S211; Yes), the controller 110 determines that the current time is the recommended wake-up time, namely the timing at which the sleep state is light, and executes step S205 (alarm start). The determination condition in step S209 is one condition for determining whether the heart rate is correctly acquired in the determination period and, as such, is also called a "second condition."

As a result of the analysis processing and the determination processing described above, the controller 110 of the electronic device 100 acquires a plurality of the first data and a plurality of the second data different from the plurality of first data in the first period; sets the first threshold on the basis of the acquired plurality of first data; sets the second threshold on the basis of the acquired plurality of second data; and determines the sleep state of the user on the basis of the determination data acquired in the second period different from the first period, and at least one threshold of the first threshold or the second threshold. Accordingly, the controller 110 can determine, as the recommended wake-up time, the timing at which the sleep state of the user is light. Moreover, it is possible to wake up the user by starting up the alarm at that timing.

As described above, the controller 110 can remove the two types of outliers (suddenly occurring outliers and continuously occurring outliers) that occur in the heart rate data. Specifically, the suddenly occurring outliers can be removed by the Min filter, and the continuously occurring outliers can be removed by using the standard deviations (for example, by using the standard deviations and the average of the heart rate corresponding to times at which the standard deviation is less than or equal to the 25th percentile value). Accordingly, the controller 110 can more appropriately determine the sleep state of the user.

Additionally, when a determination is made that the correct heart rate is not being acquired, the controller 110 determines the sleep state of the user using the acceleration instead of the heart rate and, as such, the controller 110 can determine the sleep state of the user more appropriately than when using only the heart rate. For example, when the belt of the electronic device 100 (for example, a smartwatch) loosens while the user is sleeping, appropriate detection of the heart rate becomes impossible. Since the acceleration can be detected without issue in such a case as well, the sleep state can be more correctly determined by using the acceleration data instead of the heart rate data.

Additionally, when the controller 110 determines that the heart rate (the first data) cannot be appropriately acquired (the first condition is not satisfied) in the analysis period (the first period), the controller 110 determines, in the determination period (the second data), the sleep state of the user on the basis of the acceleration (the second data) and the acceleration threshold without using the heart rate threshold that is thought to have low reliability. As such, the controller 110 can more correctly determine the sleep state.

Moreover, even when the controller 110 determines that the heart rate (the first data) has been correctly acquired in the analysis period (the first period), when it is thought that, in the determination period (the second period) the reliability of the heart rate data acquired as the determination data is low (when the second condition is not satisfied), the controller 110 determines the sleep state of the user on the basis of the acceleration (the second data) and the acceleration threshold without using the heart rate (the first data). As such, the controller 110 can more correctly determine the sleep state.

### Modified Example 1

In the determination processing (FIG. 13) according to Embodiment 1 described above, in step S209, the possibility of the heart rate being outside the predetermined reference value range is considered. However, in actuality, the possibility of being outside the predetermined reference value range is extremely small and, as such, in the analysis processing (FIG. 12), processing that takes this possibility into consideration is not performed. However, as Modified Example 1, a configuration is possible in which processing that takes this possibility into consideration is performed.

In Modified Example 1, when the heart rate calculated in step S103 is outside the predetermined reference value range (is less than or equal to the abnormal determination threshold), the controller 110 considers that heart rate as an outlier and ignores it. By configuring in this manner, in Modified Example 1, even if an (abnormally small) outlier outside the predetermined reference value range occurs suddenly as a heart rate in the analysis period, it is possible to set a heart rate threshold in which the effects of that outlier are eliminated as much as possible.

### Modified Example 2

In the analysis processing (FIG. 12) and the determination processing (FIG. 13) according to Embodiment 1 described above, the Min filter is used to remove outliers such as the heart rate suddenly becoming a large value. However, with some algorithms for calculating the heart rate from the pulse wave, very few outliers, such as the heart rate suddenly becoming a large value, occur and, conversely, there are comparatively many cases in which outliers, such as the heart rate becoming a small value, occur. When adopting such an algorithm, it is desirable to use a Max filter that replaces the heart rate with the maximum value within the most recent predetermined period (window), instead of the Min filter.

In this Modified Example 2, in step S209 of the determination processing (FIG. 13), a determination is made whether the maximum value of the heart rate is within the predetermined reference value range (whether smaller than the abnormal determination threshold (for example, 200)). By using the Max filter, in Modified Example 2, even if small value outliers suddenly occur as heart rates, it is possible to perform the setting of the heart rate threshold and the determination of the recommended wake-up time in which the effects of those outliers are eliminated as much as possible.

### Modified Example 3

With some algorithms for calculating the heart rate from the pulse wave, there are cases in which the heart rate suddenly takes large values and small values. When adopting such an algorithm, it is desirable to use a Median filter that replaces the heart rate with the median within the most recent predetermined period (window), instead of the Min filter and/or the Max filter.

In Modified Example 3 described above, in step S209 of the determination processing (FIG. 13), two types of abnormal determination thresholds (first abnormal determination threshold and second abnormal determination threshold) are prepared, and a determination is made whether the heart rate is between the first abnormal determination threshold (for example, 10) and the second abnormal determination threshold (for example, 200). By using the Median filter, in Modified Example 3, even if small value outliers and/or large value outliers suddenly occur as heart rates, it is possible to perform the setting of the heart rate threshold and the determination of the recommended wake-up time in which the effects of those outliers are eliminated as much as possible.

### Other Modified Examples

The present disclosure is not limited to the embodiments described above, and various modifications and uses are possible. For example, in the embodiment and the modified examples described above, the sensor 130 includes a pulse wave sensor for detecting the pulse wave and an acceleration sensor for detecting the body motion. However, in an unclaimed embodiment, the types of sensors that the sensor 130 is provided with are not limited thereto. The sensor 130 can include any type of sensor that can detect (acquire) a biological signal to be used in the determination of the recommended wake-up time. Additionally, in a case in which the biological signals or feature values for estimating the sleep state can be received from an external device or the like via the communicator 160, for example, the electronic device 100 need not include the sensor 130, and the biological signal or the like may be acquired via the communicator 160.

The electronic device 100 is not limited to a wearable device such as a smartwatch. A configuration is possible in which the electronic device 100 is an information device such as a smartphone that acquires, via wireless communication or the like, the heart rate and/or the acceleration of the user from another wearable terminal or the like (a smartwatch or the like). In such a case, a configuration is possible in which the analysis processing and the determination processing are carried out by the electronic device 100 serving as the information device, and the alarm function is carried out by the information device main body; or a configuration is possible in which alarm information is sent to the wearable terminal and the alarm sound is output by the wearable terminal.

**In** the embodiment and the modified examples described above, the electronic device 100 uses the heart rate (first data) and the acceleration (second data) as the biological signal data to be used in the determination of the sleep state of the user. However, in an unclaimed embodiment, the biological signals to be used in the determination are not limited thereto. Examples of biological signals usable by the electronic device 100 include body motion (detected by an acceleration sensor provided on the head, arm, chest, foot, torso, or the like), EMG (detected by myoelectric sensors attached to the head (around the temples and nose), arms, chest, feet, torso, or the like), perspiration (detected by a skin electrometer or a humidity sensor), heartbeat (detection by an electrocardiograph, a pressure sensor installed under a bed (detection of ballistocardiogram waveform), a pulse wave sensor installed on the head, arm, chest, feet, torso, or the like), and the like.

Note that, in the embodiments and the modified examples described above, the electronic device 100 includes the input device 140, the output device 150, and the communicator 160. However, these are not essential constituents and configurations are possible in which the electronic device 100 does not include the input device 140, the output device 150, and/or the communicator 160.

The various functions of the electronic device 100 can be implemented by a computer such as a typical personal computer (PC). Specifically, in the embodiments described above, examples are described in which programs, such as the analysis processing, executed by the electronic device 100 are stored in advance in the ROM of the storage 120. However, a computer may be configured that is capable of realizing these various features by storing and distributing the programs on a non-transitory computer-readable recording medium such as a compact disc read-only memory (CD-ROM), a digital versatile disc (DVD), a magneto-optical disc (MO), a memory card, and universal serial bus (USB) memory, and reading out and installing these programs on the computer.

Additionally, in cases in which the processings described above are realized by being divided between an operating system (OS) and an application/program, or are realized by cooperation between an OS and an application/program, it is possible to store only the portion of the application/program on the non-transitory recording medium or in the storage. Additionally, the programs can be piggybacked on carrier waves and distributed via a network. For example, the programs may be posted to a bulletin board system (BBS) on a network, and distributed via the network. Moreover, a configuration is possible in which the processings described above are executed by starting these programs and, under the control of the operating system (OS), executing the programs in the same manner as other applications/programs.

Additionally, a configuration is possible in which the controller 110 is constituted by a desired processor unit such as a single processor, a multiprocessor, a multi-core processor, or the like, or by combining these desired processors with processing circuity such as an application specific integrated circuit (ASIC), a field-programmable gate array (FPGA), or the like.

The foregoing describes some example embodiments for explanatory purposes. Accordingly, the specification and drawings are to be regarded in an illustrative rather than a restrictive sense. This detailed description, therefore, is not to be taken in a limiting sense, and the invention is only defined by the scope of the appended claims.

## Claims

1. An electronic device (100) comprising:
a sensor (130) that includes a pulse wave sensor configured to acquire heart rate data of a user and an acceleration sensor for detecting body motion of the user; and
a controller (110) configured to determine a sleep state of a user, based on first data and second data different from the first data, the first data including data relating to a heart rate, the second data including data detected by an acceleration sensor and relating to a body motion,
the controller (110) being further configured to acquire, as data for setting thresholds, a plurality of the first data and a plurality of the second data in a first period,
set a first threshold based on the acquired plurality of first data,
set a second threshold based on the acquired plurality of second data,
determine whether the first data acquired in the first period satisfies a first condition,
when a determination is made that the first condition is not satisfied, determine the sleep state of the user, based on determination data that is the second data acquired in a second period different from the first period, and the second threshold, and
when a determination is made that the first data acquired in the first period satisfies the first condition, determine whether the first data acquired in the second period satisfies a second condition,
when a determination is made that the second condition is not satisfied, determine the sleep state of the user, based on the second data acquired in the second period and the second threshold, and
when a determination is made that the second condition is satisfied, determine the sleep state of the user, based on the first data acquired in the second period and the first threshold.

2. The electronic device (100) according to claim 1, wherein the first condition is that a standard deviation of the first data acquired in the first period is included in a predetermined range.

3. The electronic device (100) according to claim 1, wherein the second condition is that minimum value within a first window period of the first data acquired in the second period is greater than an abnormal determination threshold.

4. A sleep state determination method to be executed by a controller (110) of an electronic device (100) comprising a sensor (130) that includes a pulse wave sensor configured to acquire heart rate data of a user and an acceleration sensor for detecting body motion of the user, the method comprising:
determining a sleep state of a user by the controller (110), based on first data and second data different from the first data, the first data including data relating to a heart rate, the second data including data detected by an acceleration sensor and relating to a body motion,
acquiring, as data for setting thresholds, a plurality of the first data and a plurality of the second data by the controller (110) and in a first period;
setting a first threshold by the controller (110) and based on the acquired plurality of first data;
setting a second threshold by the controller (110) and based on the acquired plurality of second data; and
determining whether the first data acquired in the first period satisfies a first condition by the controller (110),
when a determination is made that the first condition is not satisfied, determining the sleep state of the user, based on determination data that is the second data acquired in a second period different from the first period, and the second threshold,
when a determination is made that the first data acquired in the first period satisfies the first condition, determining whether the first data acquired in the second period satisfies a second condition,
when a determination is made that the second condition is not satisfied, determining the sleep state of the user, based on the second data acquired in the second period and the second threshold, and
when a determination is made that the second condition is satisfied, determining the sleep state of the user, based on the first data acquired in the second period and the first threshold.

5. The sleep state determination method according to claim 4, wherein the first condition is that a standard deviation of the first data acquired in the first period is included in a predetermined range.

6. The sleep state determination method according to claim 4, wherein the second condition is that minimum value within a first window period of the first data acquired in the second period is greater than an abnormal determination threshold.

7. A program executable by at least one controller (110) of a computer of an electronic device (100) comprising a sensor (130) that includes a pulse wave sensor configured to acquire heart rate data of a user and an acceleration sensor for detecting body motion of the user, the program causing the at least one controller (110) to carry out the following method steps, in accordance with the program:
determining a sleep state of a user, based on first data and second data different from the first data, the first data including data relating to a heart rate, the second data including data detected by an acceleration sensor and relating to a body motion,
acquiring, as data for setting thresholds, a plurality of the first data and a plurality of the second data in a first period;
setting a first threshold based on the acquired plurality of first data;
setting a second threshold based on the acquired plurality of second data; and
determining whether the first data acquired in the first period satisfies a first condition,
when a determination is made that the first condition is not satisfied, determining the sleep state of the user, based on determination data that is the second data acquired in a second period different from the first period, and the second threshold,
when a determination is made that the first data acquired in the first period satisfies the first condition, determining whether the first data acquired in the second period satisfies a second condition,
when a determination is made that the second condition is not satisfied, determining the sleep state of the user, based on the second data acquired in the second period and the second threshold, and
when a determination is made that the second condition is satisfied, determining the sleep state of the user, based on the first data acquired in the second period and the first threshold.

8. The program according to claim 7, wherein the first condition is that a standard deviation of the first data acquired in the first period is included in a predetermined range.

9. The program according to claim 7, wherein the second condition is that minimum value within a first window period of the first data acquired in the second period is greater than an abnormal determination threshold.

## Patentansprüche

1. Elektronische Vorrichtung (100), umfassend:
einen Sensor (130), der einen Pulswellensensor einschließt, der ausgebildet ist, um Herzfrequenzdaten eines Benutzers zu erfassen, und einen Beschleunigungssensor zum Detektieren von Körperbewegungen des Benutzers; und
eine Steuereinheit (110), die ausgebildet ist zum
Bestimmen eines Schlafzustands eines Benutzers, basierend auf ersten Daten und zweiten Daten, die sich von den ersten Daten unterscheiden, wobei die ersten Daten Daten einschließen, die sich auf eine Herzfrequenz beziehen, die zweiten Daten Daten einschließen, die von einem Beschleunigungssensor erfasst werden und sich auf eine Körperbewegung beziehen,
wobei die Steuereinheit (110) weiter ausgebildet ist zum
Erfassen, als Daten zum Einstellen von Schwellenwerten, einer Vielzahl von den ersten Daten und einer Vielzahl von den zweiten Daten in einer ersten Periode,
Einstellen eines ersten Schwellenwerts, basierend auf der erfassten Vielzahl von ersten Daten,
Einstellen eines zweiten Schwellenwerts, basierend auf der erfassten Vielzahl von zweiten Daten,
Bestimmen, ob die ersten Daten, die in der ersten Periode erfasst werden, eine erste Bedingung erfüllen,
wenn eine Bestimmung gemacht wird, dass die erste Bedingung nicht erfüllt ist, Bestimmen des Schlafzustandes des Benutzers, basierend auf Bestimmungsdaten, die die zweiten Daten sind, die in einer zweiten Periode erfasst werden, die sich von der ersten Periode unterscheidet, und dem zweiten Schwellenwert, und
wenn eine Bestimmung gemacht wird, dass die ersten Daten, die in der ersten Periode erfasst werden, die erste Bedingung erfüllen, Bestimmen, ob die ersten Daten, die in der zweiten Periode erfasst werden, eine zweite Bedingung erfüllen,
wenn eine Bestimmung gemacht wird, dass die zweite Bedingung nicht erfüllt ist, Bestimmen des Schlafzustands des Benutzers, basierend auf den zweiten Daten, die in der zweiten Periode erfasst werden, und dem zweiten Schwellenwert, und
wenn eine Bestimmung gemacht wird, dass die zweite Bedingung erfüllt ist, Bestimmen des Schlafzustands des Benutzers, basierend auf den ersten Daten, die in der zweiten Periode erfasst werden, und dem ersten Schwellenwert.

2. Elektronische Vorrichtung (100) nach Anspruch 1, wobei die erste Bedingung darin besteht, dass eine Standardabweichung der ersten Daten, die in der ersten Periode erfasst werden, in einem vorbestimmten Bereich eingeschlossen ist.

3. Elektronische Vorrichtung (100) nach Anspruch 1, wobei die zweite Bedingung darin besteht, dass der minimale Wert innerhalb einer ersten Fensterperiode der ersten Daten, die in der zweiten Periode erfasst werden, größer ist als ein Anomaliebestimmungsschwellenwert.

4. Schlafzustandsbestimmungsverfahren, das von einer Steuereinheit (110) einer elektronischen Vorrichtung (100) auszuführen ist, umfassend einen Sensor (130), der einen Pulswellensensor, der ausgebildet ist, um Herzfrequenzdaten eines Benutzers zu erfassen, und einen Beschleunigungssensor zum Detektieren einer Körperbewegung des Benutzers einschließt, wbei das Verfahren Folgendes umfasst:
Bestimmen eines Schlafzustandes eines Benutzers durch die Steuereinheit (110), basierend auf ersten Daten und zweiten Daten, die sich von den ersten Daten unterscheiden, wobei die ersten Daten Daten einschließen, die sich auf eine Herzfrequenz beziehen, und die zweiten Daten Daten einschließen, die von einem Beschleunigungssensor detektiert werden und sich auf eine Körperbewegung beziehen,
Erfassen, als Daten zum Einstellen von Schwellenwerten, einer Vielzahl von den ersten Daten und einer Vielzahl von den zweiten Daten durch die Steuereinheit (110) und in einer ersten Periode;
Einstellen eines ersten Schwellenwerts durch die Steuereinheit (110) und basierend auf der erfassten Vielzahl von ersten Daten;
Einstellen eines zweiten Schwellenwertes durch die Steuereinheit (110) und basierend auf der erfassten Vielzahl von zweiten Daten; und
Bestimmen, ob die ersten Daten, die in der ersten Periode erfasst werden, eine erste Bedingung erfüllen, durch die Steuereinheit (110),
wenn eine Bestimmung gemacht wird, dass die erste Bedingung nicht erfüllt ist, Bestimmen des Schlafzustandes des Benutzers, basierend auf Bestimmungsdaten, die die zweiten Daten sind, die in einer zweiten Periode erfasst werden, die sich von der ersten Periode unterscheidet, und dem zweiten Schwellenwert,
wenn eine Bestimmung gemacht wird, dass die ersten Daten, die in der ersten Periode erfasst werden, die erste Bedingung erfüllen, Bestimmen, ob die ersten Daten, die in der zweiten Periode erfasst werden, eine zweite Bedingung erfüllen,
wenn ein Bestimmung gemacht wird, dass die zweite Bedingung nicht erfüllt ist, Bestimmen des Schlafzustands des Benutzers, basierend auf den zweiten Daten, die in der zweiten Periode erfasst werden, und dem zweiten Schwellenwert, und
wenn eine Bestimmung gemacht wird, dass die zweite Bedingung erfüllt ist, Bestimmen des Schlafzustandes des Benutzers, basierend auf den ersten Daten, die in der zweiten Periode erfasst werden, und dem ersten Schwellenwert.

5. Schlafzustandsbestimmungsverfahren nach Anspruch 4, wobei die erste Bedingung darin besteht, dass eine Standardabweichung der ersten Daten, die in der ersten Periode erfasst werden, in einem vorbestimmten Bereich eingeschlossen ist.

6. Schlafzustandsbestimmungsverfahren nach Anspruch 4, wobei die zweite Bedingung darin besteht, dass der minimale Wert innerhalb einer ersten Fensterperiode der ersten Daten, die in der zweiten Periode erfasst werden, größer ist als ein Anomaliebestimmungsschwellenwert.

7. Programm, das von mindestens einer Steuereinheit (110) eines Computers einer elektronischen Vorrichtung (100) ausführbar ist, umfassend einen Sensor (130), der einen Pulswellensensor, der ausgebildet ist, Herzfrequenzdaten eines Benutzers zu erfassen, und einen Beschleunigungssensor zum Detektieren von Körperbewegungen des Benutzers einschließt, wobei das Programm die mindestens eine Steuereinheit (110) veranlasst, in Übereinstimmung mit dem Programm die folgenden Verfahrensschritte auszuführen:
Bestimmen eines Schlafzustandes eines Benutzers, basierend auf ersten Daten und zweiten Daten, die sich von den ersten Daten unterscheiden, wobei die ersten Daten Daten einschließen, die sich auf eine Herzfrequenz beziehen, die zweiten Daten Daten einschließen, die von einem Beschleunigungssensor detektiert werden und sich auf eine Körperbewegung beziehen,
Erfassen, als Daten zum Einstellen von Schwellenwerten, einer Vielzahl von den ersten Daten und einer Vielzahl von den zweiten Daten in einer ersten Periode;
Einstellen eines ersten Schwellenwerts, basierend auf der erfassten Vielzahl von ersten Daten;
Einstellen eines zweiten Schwellenwerts, basierend auf der erfassten Vielzahl von zweiten Daten; und
Bestimmen, ob die ersten Daten, die in der ersten Periode erfasst werden, eine erste Bedingung erfüllen,
wenn eine Bestimmung gemacht wird, dass die erste Bedingung nicht erfüllt ist, Bestimmen des Schlafzustands des Benutzers, basierend auf Bestimmungsdaten, die die zweiten Daten sind, die in einer zweiten Periode erfasst wurden, die sich von der ersten Periode unterscheidet, und dem zweiten Schwellenwert,
wenn eine Bestimmung gemacht wird, dass die ersten Daten, die in der ersten Periode erfasst werden, die erste Bedingung erfüllen, Bestimmen, ob die ersten Daten, die in der zweiten Periode erfasst werden, eine zweite Bedingung erfüllen,
wenn eine Bestimmung gemacht wird, dass die zweite Bedingung nicht erfüllt ist, Bestimmen des Schlafzustands des Benutzers, basierend auf den zweiten Daten, die in der zweiten Periode erfasst werden, und dem zweiten Schwellenwert, und
wenn eine Bestimmung gemacht wird, dass die zweite Bedingung erfüllt ist, Bestimmen des Schlafzustandes des Benutzers, basierend auf den ersten Daten, die in der zweiten Periode erfasst werden, und dem ersten Schwellenwert.

8. Programm nach Anspruch 7, wobei die erste Bedingung darin besteht, dass eine Standardabweichung der ersten Daten, die in der ersten Periode erfasstwerden, in einem vorbestimmten Bereich eingeschlossen ist.

9. Programm nach Anspruch 7, wobei die zweite Bedingung darin besteht, dass der minimale Wert innerhalb einer ersten Fensterperiode der ersten Daten, die in der ersten Periode erfasst werden, größer ist als ein Anomaliebestimmungsschwellenwert.

## Revendications

1. Dispositif électronique (100) comprenant :
un capteur (130) qui inclut un capteur d'ondes de pouls configuré pour acquérir des données de fréquence cardiaque d'un utilisateur et un capteur d'accélération destiné à détecter un mouvement corporel de l'utilisateur ; et
une unité de commande (110) configurée pour
déterminer un état de sommeil d'un utilisateur, sur la base de premières données et de secondes données différentes des premières données, les premières données incluant des données relatives à une fréquence cardiaque, les secondes données incluant des données détectées par un capteur d'accélération et relatives à un mouvement corporel,
l'unité de commande (110) étant en outre configurée pour :
acquérir, en tant que données servant à établir des seuils, une pluralité des premières données et une pluralité des secondes données pendant une première période,
établir un premier seuil sur la base de la pluralité acquise de premières données,
établir un second seuil sur la base de la pluralité acquise de secondes données,
déterminer si les premières données acquises pendant la première période remplissent une première condition,
lorsqu'il est déterminé que la première condition n'est pas remplie, déterminer l'état de sommeil de l'utilisateur, sur la base de données de détermination qui consistent en les secondes données acquises pendant une seconde période différente de la première période, et du second seuil, et
lorsqu'il est déterminé que les premières données acquises pendant la première période remplissent la première condition, déterminer si les premières données acquises pendant la seconde période remplissent une seconde condition,
lorsqu'il est déterminé que la seconde condition n'est pas remplie, déterminer l'état de sommeil de l'utilisateur, sur la base des secondes données acquises pendant la seconde période, et du second seuil, et
lorsqu'il est déterminé que la seconde condition est remplie, déterminer l'état de sommeil de l'utilisateur, sur la base des premières données acquises pendant la seconde période, et du premier seuil.

2. Dispositif électronique (100) selon la revendication 1, dans lequel la première condition est qu'un écart-type des premières données acquises pendant la première période soit inclus dans une plage préétablie.

3. Dispositif électronique (100) selon la revendication 1, dans lequel la seconde condition est que la valeur minimale à l'intérieur d'une première période fenêtre des premières données acquises pendant la seconde période soit supérieure à un seuil de détermination d'anomalie.

4. Procédé de détermination d'état de sommeil destiné à être exécuté par une unité de commande (110) d'un dispositif électronique (100) comprenant un capteur (130) qui inclut un capteur d'ondes de pouls configuré pour acquérir des données de fréquence cardiaque d'un utilisateur et un capteur d'accélération destiné à détecter un mouvement corporel de l'utilisateur, le procédé comprenant les étapes consistant à :
déterminer un état de sommeil d'un utilisateur par l'unité de commande (110), sur la base de premières données et de secondes données différentes des premières données, les premières données incluant des données relatives à une fréquence cardiaque, les secondes données incluant des données détectées par un capteur d'accélération et relatives à un mouvement corporel,
acquérir, en tant que données servant à établir des seuils, une pluralité des premières données et une pluralité des secondes données par l'unité de commande (110) et pendant une première période ;
établir un premier seuil par l'unité de commande (110) et sur la base de la pluralité acquise de premières données ;
établir un second seuil par l'unité de commande (110) et sur la base de la pluralité acquise de secondes données, et
déterminer par l'unité de commande (110) si les premières données acquises pendant la première période remplissent une première condition,
lorsqu'il est déterminé que la première condition n'est pas remplie, déterminer l'état de sommeil de l'utilisateur, sur la base de données de détermination qui consistent en les secondes données acquises pendant une seconde période différente de la première période, et du second seuil,
lorsqu'il est déterminé que les premières données acquises pendant la première période remplissent la première condition, déterminer si les premières données acquises pendant la seconde période remplissent une seconde condition,
lorsqu'il est déterminé que la seconde condition n'est pas remplie, déterminer l'état de sommeil de l'utilisateur, sur la base des secondes données acquises pendant la seconde période, et du second seuil, et
lorsqu'il est déterminé que la seconde condition est remplie, déterminer l'état de sommeil de l'utilisateur, sur la base des premières données acquises pendant la seconde période, et du premier seuil.

5. Procédé de détermination d'état de sommeil selon la revendication 4, dans lequel la première condition est qu'un écart-type des premières données acquises pendant la première période soit inclus dans une plage préétablie.

6. Procédé de détermination d'état de sommeil selon la revendication 4, dans lequel la seconde condition est que la valeur minimale à l'intérieur d'une première période fenêtre des premières données acquises pendant la seconde période soit supérieure à un seuil de détermination d'anomalie.

7. Programme exécutable par au moins une unité de commande (110) d'un calculateur d'un dispositif électronique (100) comprenant un capteur (130) qui inclut un capteur d'ondes de pouls configuré pour acquérir des données de fréquence cardiaque d'un utilisateur et un capteur d'accélération destiné à détecter un mouvement corporel de l'utilisateur, le programme amenant ladite au moins une unité de commande (110) à mettre en œuvre les étapes de procédé suivantes, conformément au programme, consistant à :
déterminer un état de sommeil d'un utilisateur, sur la base de premières données et de secondes données différentes des premières données, les premières données incluant des données relatives à une fréquence cardiaque, les secondes données incluant des données détectées par un capteur d'accélération et relatives à un mouvement corporel,
acquérir, en tant que données servant à établir des seuils, une pluralité des premières données et une pluralité des secondes données pendant une première période ;
établir un premier seuil sur la base de la pluralité acquise de premières données ;
établir un second seuil sur la base de la pluralité acquise de secondes données ; et
déterminer si les premières données acquises pendant la première période remplissent une première condition,
lorsqu'il est déterminé que la première condition n'est pas remplie, déterminer l'état de sommeil de l'utilisateur, sur la base de données de détermination qui consistent en les secondes données acquises pendant une seconde période différente de la première période, et du second seuil,
lorsqu'il est déterminé que les premières données acquises pendant la première période remplissent la première condition, déterminer si les premières données acquises pendant la seconde période remplissent une seconde condition,
lorsqu'il est déterminé que la seconde condition n'est pas remplie, déterminer l'état de sommeil de l'utilisateur, sur la base des secondes données acquises pendant la seconde période, et du second seuil, et
lorsqu'il est déterminé que la seconde condition est remplie, déterminer l'état de sommeil de l'utilisateur, sur la base des premières données acquises pendant la seconde période, et du premier seuil.

8. Programme selon la revendication 7, dans lequel la première condition est qu'un écart-type des premières données acquises pendant la première période soit inclus dans une plage préétablie.

9. Programme selon la revendication 7, dans lequel la seconde condition est que la valeur minimale à l'intérieur d'une première période fenêtre des premières données acquises pendant la seconde période soit supérieure à un seuil de détermination d'anomalie.
